# EUROPEAN PATENT APPLICATION

(11) **EP 3 485 924 A1**
(43) Date of publication of application: **22.05.2019**
(21) Application number: 17201984.6
(22) Date of filing: 16.11.2017
(51) Int. Cl.: A61M 1/10, A61L 27/50

(54) **TOPOLOGICALLY STRUCTURED ELASTOMERIC MEMBRANES IN PARTICULAR FOR USE IN VENTRICULAR ASSIST DEVICES**

(71) Applicant: ETH Zürich, 8092 Zürich (CH)
(72) Inventor: Ferrari, Aldo, 8057 Zürich (CH); Bachmann, Björn Johann, 6003 Luzern (CH); Stefopoulos, Georgios, 8050 Zürich (CH); Bernardi, Laura, 8003 Zürich (CH); Giampietro, Costanza, 8057 Zürich (CH); Poulikakos, Dimos, 8702 Zollikon (CH); Hopf, Raoul, 3011 Bern (CH)
(74) Representative: Bremi, Tobias Hans

(57) **Abstract**

Membrane element having, on at least one side thereof, on a flat surface (1) a topological structured in the form of a two-dimensional array of regular hexagons (6) formed by walls (2) extending between a foot (5) at the flat base surface (1) and a top (3), and having a wall height (wh) perpendicular to the flat surface (1) and a wall width (ww) measured parallel to the flat surface and perpendicular to the respective wall direction, and enclosing, with the inner edges of the foot (5) of the walls, a well basal surface (wbs). The wall height (wh) according to the invention is in the range of 3-10 µm, the wall width (ww) is in the range of 2-8 µm, and the wall basal surface (wbs) is in the range of 800-2500 µm².

## Description

### TECHNICAL FIELD

The present invention relates to topologically structured membranes, in particular adapted for allowing to efficiently grow cell monolayers on top of that topological structure, for example confluent endothelial cell layers. Furthermore the invention relates to methods for making such topologically structured membranes and to uses of such topologically structured membranes, for example in the context of pulsatile ventricular assist devices.

### PRIOR ART

In 2016, heart failure affected over 37 million people worldwide. Heart transplantation, the current treatment of choice, is limited by the number of available donor organs and by patient eligibility. Thus, it is only employed in 60% of these cases. For all the others, the deployment of Ventricular Assist Devices (VADs) represents the only available lifeline. VAD operation has improved over the years, yet critical shortcomings taint their needed long-term performance. Serious adverse events are stirred by the interaction between artificial materials and blood. These include the activation of platelets and complement system that support inflammation and thrombosis, which are kept at bay by aggressive anticoagulation measures and platelet inhibition, having the serious drawback of exposing the recipient to the risk of hemorrhagic events.

The optimal protection assuring hemocompatibility in cardiovascular devices is represented by a bionic luminal surface supporting an autologous endothelium. This naturally evolved blood-compatible interface promises to ensure a riskless body integration of artificial materials. Contrary to the results obtained in animal models, the spontaneous endothelialization of implants was only occasionally reported in humans. Most experimental approaches have therefore focused on the generation of a precast endothelium able to survive upon deployment in vivo. Among these, engineered surfaces featuring rationally-designed topographies have been developed to interfere with endothelial cell (EC) adhesion hence enhancing monolayer retention under hemodynamic conditions similar to those expected in vivo.

However, the wall shear stress (WSS) estimated at the luminal surface of VADs can reach high supraphysiological values which peak well beyond the limit of EC survival. Additionally, in some device configurations, high WSS combines with cyclic wall deformation (WD) generating complex flow patterns and gradients of stress further challenging the monolayer integrity. Altogether, endothelialization strategies optimized for VADs, require significant advancements leading to the maintenance of a living, connected, and confluent endothelium under extreme loadings.

### SUMMARY OF THE INVENTION

The present invention relates to a novel, rational structuring approach aiming at supporting endothelialization at the luminal surface of VADs, consisting of organized, microfabricated arrays of continuous vertical barriers, locally generating cell-sized shelters in which WSS is significantly mitigated. The resulting interface is upscalable to cover large surfaces, applicable to soft and rigid artificial materials, and adaptable to complex architectures. Through a combined in silico and in vitro investigation we introduced, fabricated and optimized a honeycomb pattern in which the vertical and lateral feature size of topographic elements are designed to support the generation of a confluent endothelium, while ensuring maximal WSS reduction on all individual cells in the monolayer. The resulting metamaterial is tested under dynamic culturing conditions reproducing supraphysiological WSS alone or in combination with cyclic WD. Our results demonstrate the realization of adaptive remodeling of ECs interacting with honeycombs, yielding endothelial monolayers with superior cell density, resistance to flow and deformation, and facture toughness. More specifically, the present invention relates to a membrane element having, on at least one side thereof, on a flat surface a topological structured in the form of a two-dimensional array of regular hexagons formed by walls extending between a foot at the flat base surface and a top, and having a wall height wh perpendicular to the flat surface and a wall width ww measured parallel to the flat surface and perpendicular to the respective wall direction, and enclosing, with the inner edges of the foot of the walls, a well basal surface wbs. Surprisingly, it was found that if the parameters of this topological structure are properly selected, a confluent monolayer of sales, preferably endothelial cells, located on that topological structure can be efficiently grown and reduces the effective wall shear stress increasing the toughness of the endothelium by inhibiting wound expansion.

According to the invention, the wall height wh is chosen to be in the range of 3-10 µm. Indeed, as will be shown further below, if the wall height is smaller than the lower boundary, the wall shear stress is not sufficiently reduced. If it is larger than the upper boundary, the proper formation of a full monolayer in static conditions is not possible, leaving intercellular gaps.

According to the invention furthermore the wall width ww, measured at the top of the wall, is in the range of 2-8 µm, wherein selecting wall widths outside of this window are either not technically easily realizable if below the lower threshold, or lead to intercellular gaps is being above the upper threshold value. Typically the wall with at the foot is essentially the same as at the top, i.e. is also in the range of 2-8 µm, however it may also be somewhat larger, i.e. also slightly trapezoidal cross-section walls are possible, in this case the width at the foot portion is in the range of 10-30% larger than the width at the top.

According to the invention furthermore the wall basal surface wbs is in the range of 800-2500 µm² or 800-2000 µm². In this range the optimum number of endothelial cells can grow within one well and sufficiently adhere to the well.

According to a first preferred embodiment, the wall height wh is in the range of 4-8 µm, preferably in the range of 5-7 µm. As a matter of fact, optimum conditions were found to be at a wall height of around 6 µm.

According to yet another preferred embodiment, the wall width ww is in the range of 3-6 µm, preferably in the range of 3.5-5 µm.

The wall basal surface wbs can preferably be in the range of 800-1500 µm², preferably in the range of 900-1300 µm², most preferably in the range of 1000-1200 µm².

The flat surface layer as well as the walls forming the topological structure are preferably made as one piece from one same elastomer material. Generally speaking, the material of the walls and/or the flat surface is preferably selected to be an elastomer accessible to soft lithography including and an elastomeric biocompatible polymeric material, preferably a silicone material, particularly preferably selected on a silicone material on the basis of poly dimethyl siloxane (PDMS).

The flat surface layer, as well as preferably also the walls, in particular for the use in ventricular assist devices, consist of a material having a Young's modulus in the range of 0.02-2.5 MPa, preferably in the range of 0.05-1.5 MPa, most preferably in the range of 0.2-0.6 MPa.

The flat surface layer preferably has a thickness in the range of 10-1000 µm, preferably in the range of 100-750 µm, most preferably in the range of 200-600 µm.

At least the topologically structured side of the membrane element can be surface treated, preferably by way of plasma treatment, including oxygen plasma, sterilization, saline washing, or a combination thereof.

At least the topologically structured side of the membrane element comprises a coating, preferably a gelatin coating, having a thickness in the range of 1-10 µm, preferably in the range of 2-5 µm.

It should be noted that such a topological structure can be provided on one side or on both sides of the membrane. If for example the membrane is used as a pump membrane in a ventricular assist device, it may be desirable to have endothelial cells on both sides of the membrane since both sides are exposed to body fluids. If that is the case, corresponding to cited membranes can be made by providing corresponding negative injection mold having a topological negative structure on both sides.

The bottom surface enclosed by the walls of such a membrane can be furthermore provided with an array of ridges, which are preferably arranged as an array of linear parallel ridges or as an array of concentrical circular ridges, wherein the ridges preferably have a height in the range of 0.1-2 µm or 0.3 - 2 µm, most preferably in the range of 0.5-1.5 µm and a periodicity in the range of 0.3-2 µm, preferably in the range of 0.5-1.5 µm.

In particular for the use in a ventricular assist device the topologically structured surface can be provided with a confluent monolayer of endothelial cells.

Furthermore the present invention relates to a method for making a membrane element as described above. According to this method a normally solid negative template, preferably made using photolithographic topological structuring, is coated, preferably using spin coating or cast coating on a two-dimensional negative template or injection into a 3 dimensional negative template, with a starting material solution and/or suspension and/or mixture for the final membrane, subsequently cured and/or cross-linked, preferably during a time in the range of 12-48 hours, preferably including a room temperature phase with the temperature in the range of 20-30°C and a typically shorter elevated temperature phase with a temperature in the range of 40-80°C, preferably in the range of 50-70°C, to form the membrane element. The membrane element is subsequently removed from the negative template, if need be followed by at least one of washing, sterilization, for example using ethanol, plasma surface treatment, including oxygen plasma surface treatment and, if desired, gelatin coating of the topologically structured surface.

Such are sequence of steps can be followed, for making a membrane element with a monolayer of endothelial cells on the topological structure, by seeding endothelial cells onto the topologically structured membrane element and culturing for at least one day, preferably at least 2 days to generate a fully confluent monolayer.

Furthermore the present invention relates to the use of such a membrane, preferably of a membrane element comprising a confluent monolayer of endothelial cells, as a membrane in a biomedical device, preferably as a flexible membrane, most preferably as a membrane in a ventricular assist device.

Last but not least the present invention relates to a ventricular pulsatile assist device comprising a membrane as defined above, preferably as a pump diaphragm.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

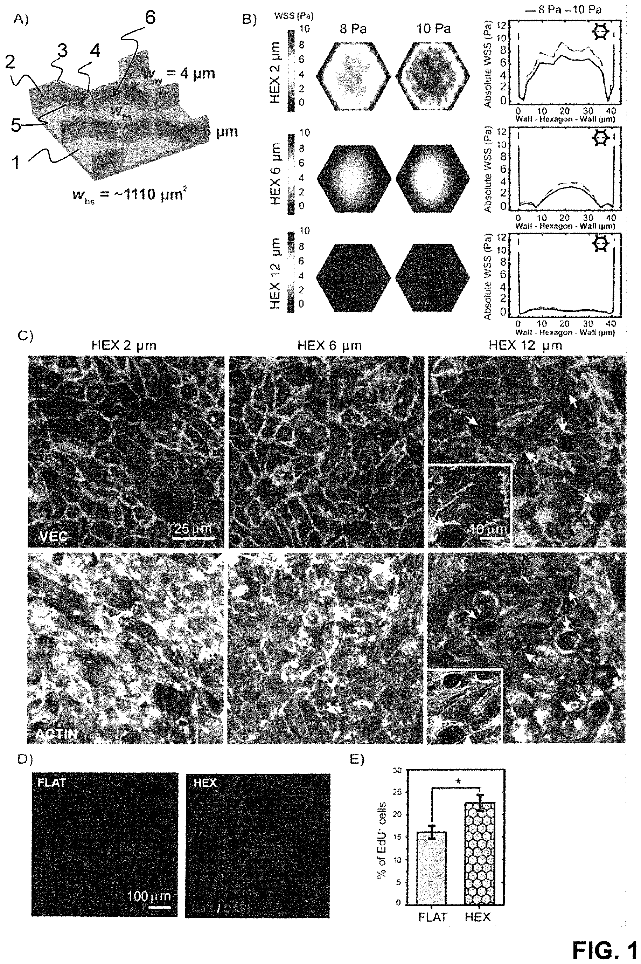

- Fig. 1: shows in A) a schematic representation of the design parameters for the hexagonal honeycomb array (HEX); in B) in silico CFD simulations of wall shear stress (WSS) distribution obtained imposing flow conditions generating nominal WSS values of 8 Pa (left column) or 10 Pa (middle column) on a flat channel with identical dimensions; the WSS mitigation generated by HEX topography with increasing height of the vertical walls (wall height Wh = 2, 6, and 12 µm; respectively) is depicted by the corresponding linear WSS profiles (right column); in C) representative immunostaining of Vascular Endothelial Cadherin (VEC) and actin of endothelial monolayers seeded on HEX topography with increasing wall height Wh of 2, 6, and 12 µm; respectively in static condition; white arrows indicate intercellular gaps; in D) EdU-proliferation assay; immunofluorescence analysis of cells on FLAT and HEX stained with EdU, distinguishing nuclei with freshly synthesized DNA from non-proliferating ones; in E) quantification of EdU-positive ECs for the proliferation assay, * = p < 0.05; n=10, n'=3;

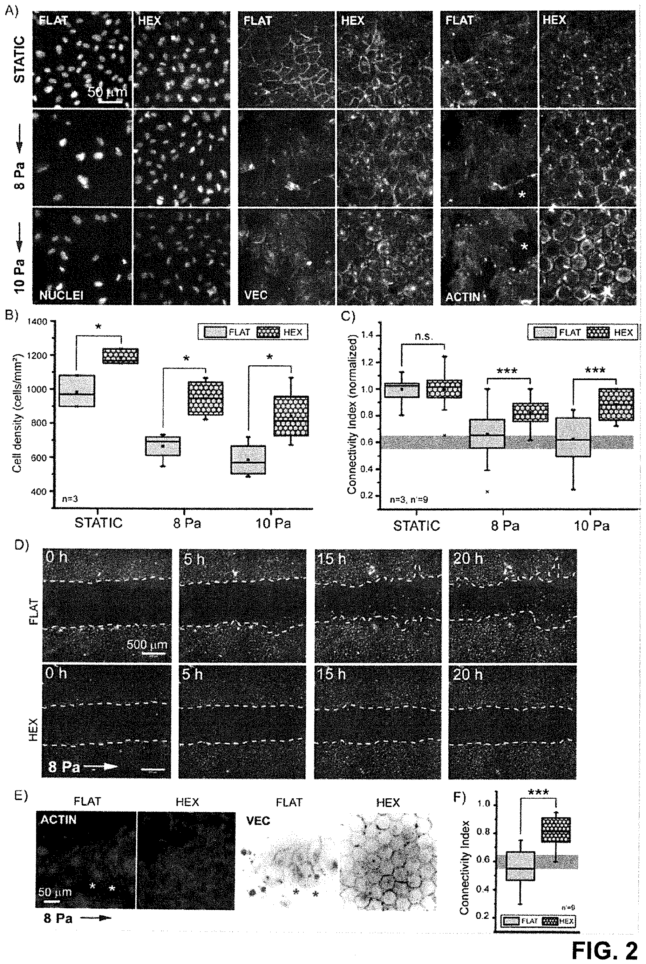

- Fig. 2: shows in a A) an immunofluorescence analysis of ECs on control flat substrates (FLAT) or hexagonal honeycomb arrays (HEX) in control static conditions or subjected to flow generating a WSS value of 8 Pa or 10 Pa on a flat channel with identical dimensions; nuclei (left panel), Vascular Endothelial Cadherin (VEC; middle panel) and actin (right panel) staining are reported; arrows indicate the direction of flow, asterisks highlight areas of substrate denudation; in B) the quantification of cell density for cells interacting with FLAT or HEX, respectively. * = p < 0.05; in C) corresponding quantification of the Connectivity Index in monolayers on FLAT or HEX substrates (*** = p < 0.001); the horizontal gray bar identifies the threshold value measured in recently confluent endothelial monolayers; in D) bright-field images of scratch wound healing experiments of ECs seeded on FLAT or HEX topography and subjected to flow generating a nominal WSS value of 8 Pa; the arrow indicates the direction of the flow, dotted lines highlight the wound edges; in E) corresponding immunofluorescence analysis of ECs at the wound edges; actin, VEC (inverted grayscale signal), nuclei staining are reported; asterisks highlight areas of substrate denudation; in F) quantification of the Connectivity Index of ECs in correspondence of the wound edges for FLAT and HEX topography;

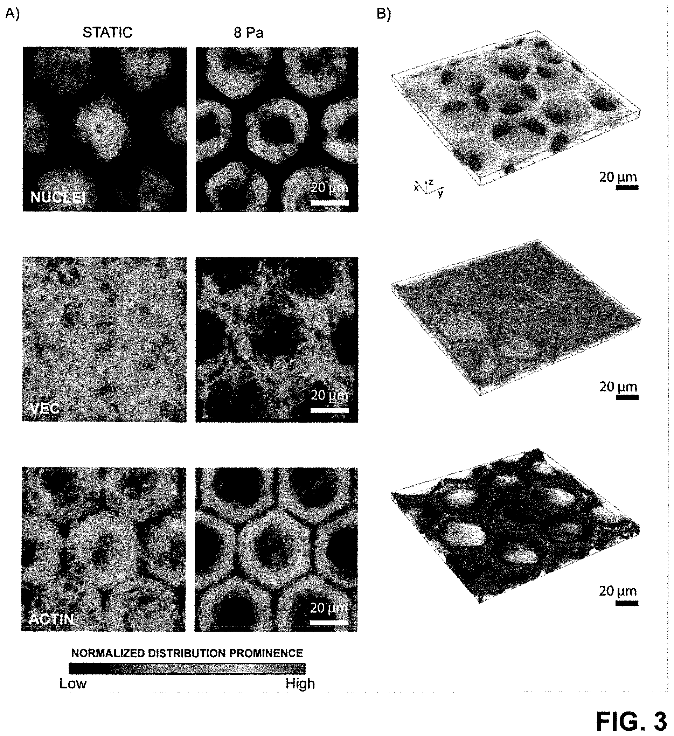

- Fig. 3: shows in A) the normalization of subcellular compartment distribution on hexagonal honeycombs; multiple images of ECs interacting with hexagonal honeycombs are collected to highlight the distribution of specific subcellular compartments; by averaging the different images the normalized or standard distribution is obtained displaying prominent localization of the cell nuclei (NUCLEI, top row), cell-to-cell junctions (VEC, middle row) and actin (ACTIN, lower row) in control static conditions (left column) or under flow generating nominal WSS values of 8 Pa (middle column) on a flat channel with identical dimensions; the number of stacked images is 25 for each condition analysed; in B) representative 3-D views of nuclei, VEC, and actin under flow obtained by volume rendering of a representative, confocal Z-stack;

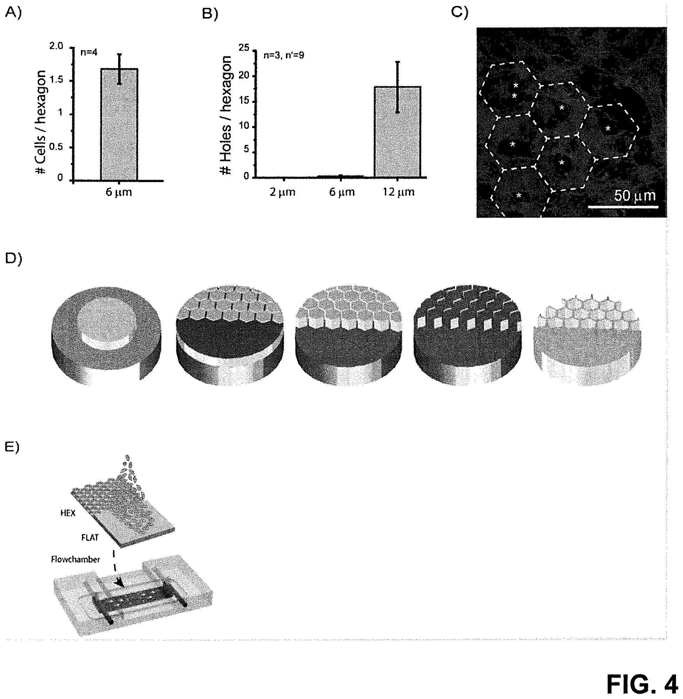

- Fig. 4: shows in A) the average number of ECs housed by individual hexagonal wells in the honeycomb array; in B) quantification of intercellular gaps in endothelial monolayers seeded on HEX topography with increasing wall height (wh = 2, 6, or 12 µm, respectively); n=6, n'=3; in C) representative immunostaining of actin in endothelial monolayers seeded on HEX topography (highlighted by dotted lines) featuring a wall height Wh = 12 µm; asterisks indicate intercellular gaps; in D) a schematic representation of the fabrication process; a Si-Wafer is spin coated with AZ6612 positive photoresist exposed to UV-light before development; the generated hexagonal honeycomb array is then transferred to the wafer in a Bosch process, and the wafer is used as a negative for hot embossing of COC; in E) a representative scheme of the experimental setup; an endothelialized patch of COC featuring both FLAT and HEX areas is mounted in a parallel plate flow chamber;

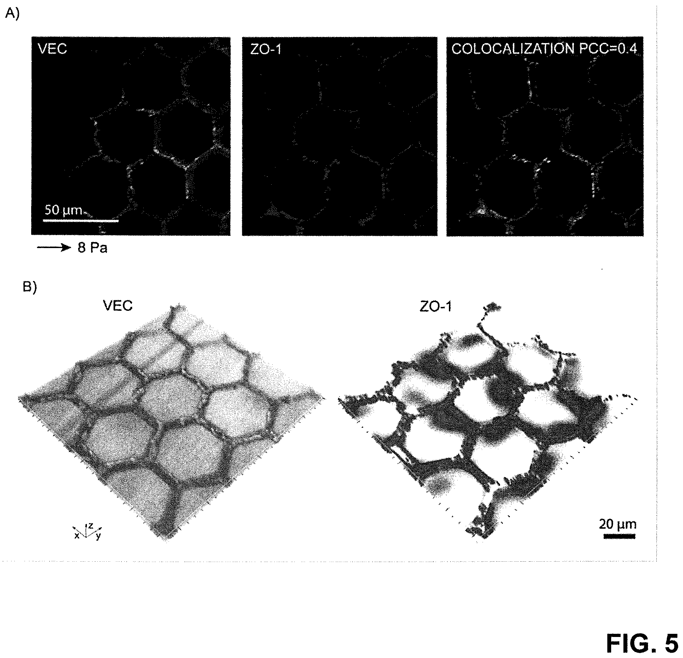

- Fig. 5: shows in A) maximum projections of fluorescent Z-stacks reporting the localization of VEC and ZO-1 staining as well as the colocalization channel for ECs seeded on HEX and subjected to flow generating nominal WSS values of 8 Pa. PCC of colocalization is 0.4; in B) 3-D volume rendered views for the VEC and ZO-1 signal;

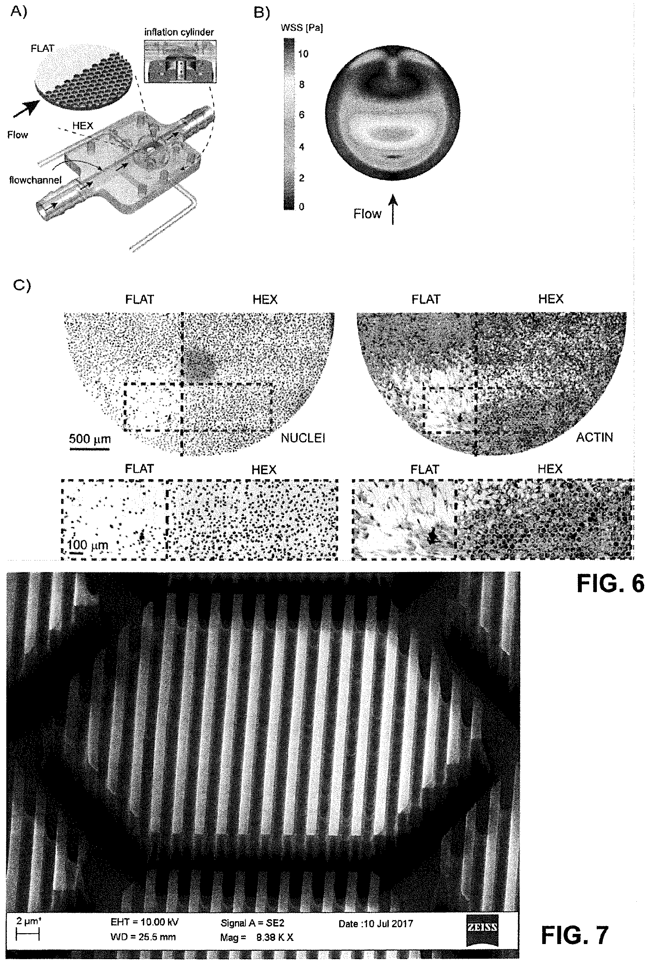

- Fig. 6: shows in a A) a cartoon depicting the experimental setup for endothelialization experiments in the combined bioreactor; elastomeric membranes featuring control flat (FLAT) and hexagonal honeycombs (HEX) aligned to the direction of flow are mounted in the bioreactor and subjected to cyclic deformation following the protocol reported in Bachmann et al. Sci Rep, 6 (2016) 38861; in B) in silico CDF simulation of the corresponding WSS profile generated during the phases of membrane inflation; in C) endpoint immunofluorescence analysis of EC retention. Nuclei (left panel), and actin (right panel) staining are reported;
- Fig. 7: shows a negative template (SEM image) for molding or hot embossing hexagonal tiling with gratings on the bottom.

### DESCRIPTION OF PREFERRED EMBODIMENTS

A hexagonal honeycomb cell pattern (HEX) was selected as initial input for the generation of a continuous, periodic, vertical wall landscape formed by perfectly-packed 3D shallow wells (see Fig. 1A) at the bottom of which the WSS from a parallel sweeping flow above, is significantly reduced. Honeycombs are optimized space-filling geometries forming an isotropic structure described by the following design parameters (see Fig.1A): wall height (wh), wall width (ww), and well basal surface (wbs). In silico CFD simulations were performed as first step to evaluate the WSS reduction at the bottom of the hexagonal wells as a function of wh (see Fig.1B). For this evaluation the value of wbs was set to 1045 µm² (see Fig.1A; hexagon outer radius r of 20 µm) corresponding to the typical contact area measured for individual human ECs in a confluent monolayer.

Considering the limits imposed by lithographic microfabrication methods ww was set to 4 µm which was comfortably realizable (Fig.1A). For the same reason the highest tested value of wh did not exceed 12 µm.

To evaluate the shielding effect of the vertical walls, the modeled geometries were exposed to a simulated flow yielding nominal WSS values of 8 or 10 Pa with respect to a completely flat and parallel channel of the same dimension. In these settings, isotropic WSS gradients were generated at the bottom of the wells (see Fig. 1B) by the interaction between the flow and the vertical barriers. Minimal WSS values (approaching 0 Pa) were found in a ring juxtaposed to the walls indicated flow stagnation, while peak values were obtained in the well center region. The difference between the nominal (i.e. imposed) and the peak (computed in the well center) WSS values, provides a conservative measure of WSS mitigation. Shallow hexagons featuring a wh of 2 µm yielded a WSS mitigation of 2-6%. This value increased to 57-59% when walls were elevated to 6 µm. The shielding effect saturated when imposing a wh of 12 µm (95%; see Fig.1B).

Based on the above guiding findings, large surfaces (12.5 mm²) featuring hexagonal honeycomb arrays with corresponding wh (2, 6, and 12 µm ± 0.05 µm) and wbs (1045 µm² ± 0.06 µm²) were fabricated on thermoplastic polymers and tested for their ability to support a fully-differentiated and confluent human endothelium.

In static conditions, ECs adhered to all tested substrates and each honeycomb housed on the average 1.7 cells (see Fig. 4A). Upon incubation, fully confluent monolayers formed on shallow structures (i.e. wh = 2 µm) in all identical to those obtained on flat controls. Here, a continuous lining of cell-to-cell junctions, the hallmark of a differentiated endothelium, was revealed by immunofluorescence staining of Vascular Endothelial Cadherin (VEC, see Fig. 1C). In addition, a homogenous surface coverage was reported by the actin signal (Fig. 1C). The same level of integrity and coverage was obtained for monolayers grown on deeper arrays (wh = 6 µm). Importantly, static cultures of ECs on 12 µm deep honeycombs never formed fully connected monolayers.

On this topography, the VEC localization showed multiple discontinuities (Fig.1C). The analysis of actin signal distribution confirmed the presence of circular holes mostly located in correspondence of the well center (Fig. 4B, C). These results indicate that, in static conditions, confluent endothelial monolayers can be formed on hexagonal honeycomb arrays featuring wh up to 6 µm while higher barriers compromise the tissue integrity. Thus, they set the maximal viable WSS mitigation to approximately 60% for a connected and continuous endothelium.

To evaluate their performance, endothelia were then grown on the selected hexagonal honeycomb array (nominal values of wh = 6 µm, ww = 4 µm, and wbs = 1045 µm²; Fig. 1A) and compared to control monolayers generated on identical flat substrates. In particular, despite the equal initial seeding, cell density was increased by 20% on topography in static conditions. This was linked to a 40% higher proliferation rate sustained by the interaction with honeycombs, as demonstrated by the DNA incorporation of 5-Ethynyl-2'-deoxyuridine (EdU) which reports on the active DNA synthesis in dividing cells (Fig. ID, E).

To evaluate their performance under realistic hemodynamic conditions, the endothelia were challenged with supraphysiological loadings in a custom-developed flow chamber (Fig. 4E). Here, ECs were exposed for 18h to flow conditions yielding nominal WSS values of 8 or 10 Pa on flat substrates. The higher density cell measured on topography in static conditions (Fig. 1D,E) was further increased to more than 40% upon exposure of endothelial monolayers to supraphysiological flow (Fig. 2A, B). This results provides a first but clear indication of an increased cell retention on structured surfaces.

To confirm this observation, we performed a complete analysis of endothelial integrity and coverage. The distribution of VEC and filamentous actin in endothelia grown on flat substrates and exposed to supraphysiological flows (8 or 10 Pa) showed a broad loss of connectivity (Fig. 2A) subtending the appearance of large open spaces between cells. This data confirm that endothelial survival is compromised under WSS higher than 6 Pa. On the contrary, the shielding effect of hexagonal honeycombs supported the retention of connected monolayers under the same flow conditions, ensuring a full coverage upon exposure to high loading as confirmed by the quantification in Fig. 2C. This data demonstrates that the contact with rationally-selected honeycomb patterns endows endothelial monolayers with superior cell density and resistance to supraphysiological wss.

We next challenged the stability of the supported endothelia upon introduction of a longitudinal wound oriented along the direction of flow. Proximity to an open space induces the directional migration of ECs aimed at the rapid re-establishment of the tissue continuity. Under supraphysiological loadings this innate behavior leads to the progressive erosion of the wound edges with a sequential wash out of individual ECs entering the wound. Therefore, on flat surfaces, the initially inflicted damage enlarges over time (Fig. 2D) in a process that rapidly compromises the tissue integrity, from the wound edges towards the rear cell rows, where a visible loss of cell-to-cell junction precedes cell detachment and substrate denudation (Fig. 2E). Longitudinal wounds with the same initial width did not widen over time on substrates structured with hexagonal honeycomb arrays (Fig. 2D, E) indicating a higher stability of the wound edges. In these experimental conditions, ECs interacting with honeycombs slowly entered the open space, while maintaining their connectivity within the monolayer, which showed a perfectly retained network of VEC cell-to-cell junctions (Fig. 2F). In all, these results demonstrate that the interaction with selected topographical patterns improves the fracture resistance of the endothelial monolayer, enhancing its stability in response to local defects and under supraphysiological hemodynamic conditions.

An in depth structural analysis showed that ECs exploited the honeycomb shelters to withstand the WSS load. As visually depicted by Fig 3, individual cells in the monolayer placed the nucleus, a mechanically-sensitive compartment in a region close to the wall thus minimizing its exposure to WSS (Fig. 3A, B upper panels).

Mechanically-resistant cell-to-cell junctions were geometrically distributed on top of the barriers (Fig. 3A, B middle panels and Fig. 5A, 2B). This multicellular architecture ensured an uncompromised endothelial integrity, which was further reinforced by the distribution of the cell cytoskeleton in a ring-like structure within the well (Fig. 3A, B lower panels). This specific arrangement cannot be purely attributed to the interaction with the basal topography, as homogenous distribution of these components was detected in static conditions. Altogether, these data provide evidence of an adaptive cellular response to the honeycomb wall topography yielding the formation of a bionic structure regulated by the WSS distribution.

Finally, the performance of surface topography in supporting a stable endothelium was extended to deformable elastomeric materials. For this purpose, the selected hexagonal honeycomb array was transferred on the surface of silicones by means of soft lithography. The resulting substrates were seeded with ECs up to the formation of a fully connected endothelium and mounted in a custom-developed combined bioreactor (according to Bachmann et al, Sci Rep, 6 (2016) 38861, see Fig. 6A). Here, the cells were subjected to a complex gradient of WSS (Fig. 6B), generated by the concomitant exposure to flow and cyclic deformation of the silicone membrane (10% WD and frequency range between 0.85 and 1.1 Hz). Specifically, the controlled actuation of membrane inflation generated an upstream region cyclically exposed to supraphysiological WSS. ECs interacting with a flat surface were gradually lost from this area (Fig. 6C) yielding a progressive local denudation of the substrate. When the membrane was symmetrically structured with the hexagonal honeycomb array (Fig 6A, 6C), the ECs loss was halted in correspondence of the transition to topography. This effect, supported by WSS mitigation and fracture toughness properties (Fig. 2B), further demonstrate the protective effect of the honeycomb wall topography under complex combinations of hemodynamic loadings, reproducing those expected in a functioning VAD.

Taken together, the collaborative approach of in silico and in vitro studies resulted in the rational generation of a protective biomedical metasurface topography supporting endothelialization under adverse realistic flow conditions. Hexagonal honeycomb array textures can be successfully implemented in a facile manner on both rigid and soft polymers to enable stable cell coverage under supraphysiological hemodynamic conditions reproducing those expected in cardiovascular implants. The presence of the metasurface texture enhances the rate of cell proliferation and guides flow so as to generate cell-sized nests in which the wall shear stress is strongly mitigated. Endothelial cells sense and adapt to this topography distributing subcellular structures as a function of their preferential exposure to hemodynamic loading, altogether hampering cell migration and supporting the maintenance of monolayer integrity under otherwise destructive conditions. These assets qualify the metasurface topography presented here as a facile and up-scalable solution to realize the long-term endothelialization of artificial implants, such as at the luminal surface of ventricular assist devices.

### Substrate fabrication.

The template wafers featuring the hexagonal honeycomb arrays were produced by standard lithography process. After fabrication, wafers were rinsed with acetone and 2-propanol and activated through an oxygen plasma treatment to facilitate silanization (15 min immersion in dimethyldichlorosilane). Surface geometries were imprinted on 180 µm thick untreated cyclic olefin copolymer (COC) foils (Ibidi, Germany) by means of nanoimprint lithography (NIL) as previously reported (see Robotti et al, Biomaterials, 35 (2014) 8479-8486). In order to transfer the same geometries to silicone elastomers the template wafer was used as direct mold. In particular, RTV components A and B (Bluestar Silicones, France, SILBIONE ® RTV 4420 A/B, the product cures by polyaddition reaction of silicone reactive groups (-SiH) with silicone unsaturated polymers including -Si-CH-CH2 groups polymerizing/cross-linking in the presence of a platinum catalyst) were mixed at a 1:1 ratio at room temperature and cast onto the template. To achieve uniform thickness of the structured molds, a custom milled Teflon plate with 400 µm spacer posts was used. Silicone membranes featuring the desired topography were then cured for 24 h at room temperature and 2 h at 60°C and carefully removed from the wafer. At the end of the fabrication procedure, all substrates were treated with oxygen plasma (100 W for 300 s), to increase the hydrophilicity of the surface for the gelatin coating (Fig. 4D). For a direct comparison within each experiment, COC and RTV with unstructured (FLAT) and topographically-modified (HEX) surfaces in the same patch were produced (Fig. 4E and Fig. 6A). The fidelity of the fabrication process was evaluated by means of Scanning Electron Microscopy (SEM).

As explained above, it is also possible to provide further topological structure of smaller dimension on the bottom surface of at least one, preferably of all hexagonal wells in the structure. How this can be done is illustrated in figure 7. The bottom surface enclosed by the walls in this case is provided with an array of parallel ridges having a width of approximately 1 µm and a periodicity of approximately 2 µm. The ridges can be, as illustrated in this figure, arranged perpendicular to one side and the opposite of the hexagon, they can however also be arranged parallel to one side and the opposite side of the hexagon.

### Cell Culture

Human umbilical vein endothelial cells (HUVECs; ThermoFisher Scientific, USA) were grown in medium M200PRF supplemented with fetal bovine serum 2% v/v, hydrocortisone 1 mg/ml, human epidermal growth factor 10 ng/ml, basic fibroblast growth factor 3 ng/ml, and heparin 10 mg/ml (all reagents from ThermoFisher Scientific) and maintained at 37°C and 5% CO2. All reported experiments were performed using cells with less than seven passages in vitro.

Surfaces for static or dynamic experiments were coated with gelatin following the protocol established by Lampugnani et al. (J. of Cell Biology Vol 129 No 1, p 203-217 (1995)). The substrates were initially sterilized by overnight treatment with ethanol and rinsed three times with PBS before starting the coating procedure. After coating the substrates were stored at 4 °C until use. Endothelial cells were seeded at high density (3.5-5 × 10⁴ cells/cm²) and cultured for three days in static conditions to generate a fully confluent and growth-arrested monolayer. (J. of Cell Science Vol 110, p 2065-2077 (1997)).

### Proliferation assay

The DNA synthesis-based cell proliferation assay was performed using a commercially-available Click-iT EdU Imaging Kits Protocol (Thermo Fisher Scientific) and following the manufacturer recommendations. After 24 h in culture, endothelial cells interacting with control flat or topographically-modified surfaces were incubated overnight with 10 µM 5-Ethynyl-2'-deoxyuridine (EdU) labeling solution, then fixed with 4% formaldehyde and permeabilized with 0.5% Triton X-100 in PBS. The samples were immediately imaged using a wide-field fluorescent microscope.

### Dynamic conditioning of Endothelial Cells

Flow experiments were performed using a custom-developed parallel plate flow bioreactor as previously described (see Franco et al, Biointerphases, 8 (2013) 34 and Robotti et al, Biomaterials, 35 (2014) 8479-8486. Endothelia grown on control flat or topographically-modified plastic substrates were mounted in the bioreactor and initially conditioned for 1 h with a flow yielding a nominal WSS of 1.4 Pa on flat surfaces. The flow-rate was then increased to reach the target WSS values of 8 or 10 Pa which were maintained for 18 h before cell fixation.

For the wound-scratch assays under flow, a longitudinal wound (average width of 1 ± 0.05 mm) was inflicted to the monolayer by means of plastic pipette tip. The endothelial were then labelled with a fluorescent dye for live cell imaging before mounting the substrate in the flow bioreactor. Care was taken to align the wound to the direction of flow.

Experiments exposing endothelial monolayers to a combination of WSS and WD were performed using an original combined bioreactor according to Bachmann et al, Sci Rep, 6 (2016) 38861. Endothelia grown on silicon membranes were mounted in the reactor flow channel. The flowrate was set to 550 ml/min resulting in a maximum shear of approximately 10 Pa and the membranes were cyclically (∼ 1 Hz) actuated to yield a maximal WD of 10% in the inflated state. Endothelial cells were exposed to combined loads for 18 h before fixation and immunostaining.

### Cell staining

The following primary antibodies were used for immunofluorescence: goat polyclonal anti-VEC (1:200, Vascular Endothelial Cadherin; #6458) from Santa Cruz Biotechnology Inc. (USA) and mouse monoclonal anti- ZO-1 (1:100, ZO1-1A12; #MA3-39100-A647) Alexa Fluor 647 conjugate from ThermoFisher Scientific. The secondary antibody was a rabbit anti-goat IgG (H+L) Alexa Fluor 488 (1:400, #A27012) conjugate from ThermoFisher Scientific (USA).

For endpoint analysis HUVECs were fixed and permeabilized for 10 min with 3% paraformaldehyde (PFA) and 0.1% Triton-X100 in PBS at room temperature (RT). The cells were then post-fixed with 3% PFA in PBS for 15 min. After washing the samples three times for 5 min with PBS, they were incubated with 5% bovine serum albumin (BSA) in PBS for 2 h at RT. The samples were incubated with TRITC-phalloidin (Sigma, USA) and primary antibody overnight at 4°C. Subsequently, the samples were rinsed four times for 1 h each with 5% BSA in PBS and then incubated with the secondary antibody for 45 min at RT. Finally, samples were washed three times (1 h each) in PBS, post-fixed for 2 min in 3% PFA, briefly washed again with PBS, mounted with Fluoroshield histology mounting medium with DAPI (Sigma, USA) and immediately imaged.

For live cell imaging of cell migration in the wound-scratch assay, cells were labelled using a Dil Vybrant Solution (Invitrogen). For the labeling, growth medium was removed and replaced with 1 mL of medium supplemented with 3 µL of the dye. The cells were then incubated for 35 min at 37°C. The staining solution was subsequently replaced with complete growth medium, before mounting the substrate in the flow bioreactor.

### Image Acquisition

The immunostained samples were imaged using an inverted Nikon-Ti spinning disk confocal microscope (Nikon, Japan) equipped with an Andor DU-888 camera (Oxford Instruments, United Kingdom) and a pE-100 LED illumination system (CoolLED Ltd, Andover, United Kingdom). Fluorescent images of immunostained HUVECs on the flat or topographically-modified substrates were acquired with a 20X, 0.75 NA air objective (Plan Apo, Nikon, Japan), using FITC, TRITC, DAPI and far red filters, for the acquisition of actin, VEC, and nuclear signals; respectively. Large images up to 6 mm × 6 mm were acquired using the Custom Multipoint/Large Image function of NIS Elements (NIS Elements, Nikon, Japan) in combination with an autofocus routine followed by automated stitching. Z-stacks for 3D volume rendering were acquired with a 60X, 1.4 NA oil objective. (Plan Apo, Nikon, Japan).

Live cell imaging of cell migration in the wound-scratch assay was obtained using a 10X, 0.3 NA, long distance objective (Plan Fluor, Nikon, Japan). The time-lapse experiment was set to routinely acquire images in the TRITC channel for multiple positions along the wound with a time resolution of 10 min for a total of 18 h. At the end of the experiment the resulting time-lapses were converted into individual 16 bit movies for post-processing.

### Image Analysis

Images were analyzed and processed using Fiji (National Institutes of Health, USA). In particular, for the evaluation of the cell density and average number of cells nested in each hexagonal well, fluorescent image of nuclear staining was obtained in a 2 mm x 2 mm area of the substrate and evaluated using the "Analyze Particles" plug-in.

To evaluate the monolayer integrity a connectivity index (CI) measurement was performed to reveal the distribution of VEC at the cell membrane. CI values close to 1 are indicative of a monolayer with a fully retained network of cell-to-cell junctions, while values close to 0 report of a diffuse VEC signal in the cytoplasm, typical of cells with disassembled or immature junctions. A threshold value is set at 0.6, corresponding to the transition between a recently confluent and a mature endothelium.

For the generation of maps reporting the average distribution prominence of subcellular compartments (see Fig. 3) multiple fluorescent images where acquired reporting the distribution of the cell nuclei, VEC at the cell-to-cell junctions, filamentous actin, and ZO-1 at the tight junctions. Regions of interest featuring unit elements of the hexagonal honeycomb array (see Fig. 1A) were cropped, registered and stacked. The stacked images were then treated as a Z-stack and averaged using the 'Average Z-projection' tool of Fiji. The fluorescent intensities were then converted to color for better visibility. 3D rendering of confocal Z-stacks (see Fig. 3B) were generated using the volume view function of NIS Elements (NIS Elements, Nikon, Japan).

### Scanning electron microscopy (SEM)

To evaluate the fine structure of endothelial monolayers interacting with hexagonal honeycombs, the substrates were fixed with 2.5% glutaraldehyde (Sigma) in sodium cacodylate buffer (pH 7.2, 0.15 M) for 1 h. Stepwise dehydration was achieved through an ethanol series of 50%, 70% and 98%. A 20 nm thick gold layer was applied to the sample prior to imaging. The substrates were then loaded into a Hitachi SU8200 SEM and image acquisition was performed.

### Computational fluid dynamics (CFD) simulations

CFD simulations were performed with COMSOL Multiphysics 5.2 (COMSOL Inc., USA) using the direct MUMPS solver. The fluid was modeled as water with an assumed molar mass M =0.0180 kg/mol, density of ρ = 997.048 kg/m3 and viscosity of µ = 8.4 x 10-4 Pa·s. A periodic element of the topography was designed with the CAD software Siemens NX 10 and imported into COMSOL. A tetrahedral mesh with a fine resolution was employed. Standard no slip conditions were used for the walls while side fluxes were assumed to equal zero due to symmetry. All simulations were performed in the EULER cluster of ETH Zurich.

### Statistics

All quantitative measurements reported are expressed as average values ± the standard error of the mean. The total number of events counted is shown in the upper or lower right hand corner of the presented graphs. The Shapiro-Wilk test was used to test for normal distribution of the data. For the normal distributed data, the paired Students t-test was employed, while for non-normal distributed data the nonparametric Mann-Whitney test was performed. For box plots the size depicts the measured standard error of the mean and the whiskers depict the 5% and 95% confidence intervals.

**LIST OF REFERENCE SIGNS**

| | | | |
|---|---|---|---|
| 1 | flat base surface | FLAT | surface without topological structuring |
| 2 | wall | | |
| 3 | top of 2 | FOV | field-of-view |
| 4 | crossing of walls | HEX | hexagonal surface topography |
| 5 | foot of 2 | HUVEC | human umbilical vein endothelial cells |
| 6 | hexagon | | |
| | | PBS | phosphate buffered saline |
| CFD | computational fluid dynamics | RTV | room temperature vulcanization |
| CI | connectivity index | | |
| COC | cyclic olefin copolymer | VAD | ventricular assist device |
| EC | endothelial cells | VEC | vascular endothelial cadherin |
| EdU | 5-Ethynyl-2'-deoxyuridine | WSS | wall shear stress parallel walls of the regular hexagon |
| r | hexagon outer radius, defined as the radius of the circle fitting into the regular hexagon and touching the 6 inner wall edges | d | hexagon width |
| | | Wh | wall height |
| | | Ww | wall width |
| | | Wbs | well basal surface |
| s | hexagon side length, defined as the distance between the inner surfaces of respectively | | |

## Claims

1. Membrane element having, on at least one side thereof, on a flat surface (1) a topological structured in the form of a two-dimensional array of regular hexagons (6) formed by walls (2) extending between a foot (5) at the flat base surface (1) and a top (3), and having a wall height (wh) perpendicular to the flat surface (1) and a wall width (ww) measured parallel to the flat surface and perpendicular to the respective wall direction, and enclosing, with the inner edges of the foot (5) of the walls, a well basal surface (wbs),
wherein the wall height (wh) is in the range of 3-10 µm,
wherein the wall width (ww) is in the range of 2-8 µm,
and wherein the wall basal surface (wbs) is in the range of 800-2500 µm².

2. Membrane element according to claim 1, wherein the wall height (wh) is in the range of 4-8 µm, preferably in the range of 5-7 µm.

3. Membrane element according to any of the preceding claims, wherein the wall width (ww) is in the range of 3-6 µm, preferably in the range of 3.5-5 µm.

4. Membrane element according to any of the preceding claims, wherein the wall basal surface (wbs) is in the range of 800-2000 µm² or 800 -1500 µm², preferably in the range of 900-1300 µm², most preferably in the range of 1000-1200 µm².

5. Membrane element according to any of the preceding claims, wherein the flat surface layer (1) as well as the walls (2) forming the topological structure are made of as one piece from one same elastomer material, which is preferably selected to be an elastomer accessible to soft lithography including a silicone material, particularly preferably selected on a silicone material on the basis of poly dimethyl siloxane (PDMS).

6. Membrane element according to any of the preceding claims, wherein the flat surface layer (1), as well as preferably also the walls (2) consist of a material having a Young's modulus in the range of 0.02-2.5 MPa, preferably in the range of 0.05-1.5 MPa, most preferably in the range of 0.2-0.6 MPa.

7. Membrane element according to any of the preceding claims, wherein the flat surface layer (1) has a thickness in the range of 10-1000 µm, preferably in the range of 100-750 µm, most preferably in the range of 200-600 µm.

8. Membrane element according to any of the preceding claims, wherein at least the topologically structured side of the membrane element is surface treated, preferably by way of plasma treatment, including oxygen plasma, sterilization, saline washing, or a combination thereof.

9. Membrane element according to any of the preceding claims, wherein at least the topologically structured side of the membrane element comprises a coating, preferably a gelatin coating, having a thickness in the range of 1-10 µm, preferably in the range of 2-5 µm.

10. Membrane element according to any of the preceding claims, wherein the bottom surface enclosed by the walls (2) is provided with an array of ridges, which are preferably arranged as an array of linear parallel ridges or as an array of concentrical circular ridges, wherein the ridges preferably have a height in the range of 0.1-2 µm, most preferably in the range of 0.3-1.5 µm or 0.5-1.5 µm and a periodicity in the range of 0.3-2 µm, preferably in the range of 0.5-1.5 µm.

11. Membrane element according to any of the preceding claims, wherein the topologically structured surface is provided with a confluent monolayer of endothelial cells.

12. Method for making a membrane element according to any of the preceding claims, wherein a solid negative template, preferably made using photolithographic topological structuring, is coated, preferably using spin coating or cast coating on a two-dimensional negative template or injection into a 3 dimensional negative template, with a starting material solution and/or suspension and/or mixture, subsequently cured and/or cross-linked, preferably during a time in the range of 12-48 hours, preferably including a room temperature phase with the temperature in the range of 20-30°C and an elevated temperature phase with a temperature in the range of 40-80°C, preferably in the range of 50-70°C, to form the membrane element which is then removed from the negative template, if need be followed by at least one of washing, sterilization, plasma surface treatment, including oxygen plasma surface treatment and gelatin coating of the topologically structured surface.

13. Method according to claim 12 for making a membrane element according to claim 11, wherein endothelial cells are seeded onto the topologically structured membrane element and cultured for at least one day, preferably at least 2 days to generate a fully confluent monolayer.

14. Use of a membrane according to any of the preceding claims 1-10, preferably of a membrane element according to claim 11 comprising a confluent monolayer of endothelial cells as a membrane in a biomedical device, preferably as a membrane, most preferably as a membrane in a ventricular assist device.

15. Ventricular pulsatile assist device comprising a membrane according to any of claims 1-11 as a pump diaphragm.
